# EUROPEAN PATENT SPECIFICATION

(11) **EP 2 152 244 B1**
(45) Date of publication and mention of the grant of the patent: **02.12.2015**
(21) Application number: 08738241.2
(22) Date of filing: 27.04.2008
(51) Int. Cl.: A61K 9/19

(54) **ORAL ADMINISTRATION OF PROTEINS AND PEPTIDES**
ORALE VERABREICHUNG VON PROTEINEN UND PEPTIDEN
ADMINISTRATION ORALE DE PROTÉINES ET DE PEPTIDES

(30) Priority: 26.04.2007 US 914134 P
(43) Date of publication of application: 17.02.2010
(73) Proprietor: Technion Research & Development Foundation Ltd., 32000 Haifa (IL)
(72) Inventor: SHIMONI, Eyal, 34368 Haifa (IL); RAMON, Ory, 30099 Sarid (IL); KOPELMAN, Isaiah J., 34404 Haifa (IL); MIZRAHI, Shimon, 32972 Haifa (IL); SALZMAN, Nir, 38363 Hadera (IL); NAHMIAS, Yaakov, Mevaseret Zion 90805 (IL); OREN, Aharon, 40300 Kfar Yonah (IL)
(74) Representative: Vossius & Partner Patentanwälte Rechtsanwälte mbB
(86) International application number: PCT/IL2008/000539
(87) International publication number: WO 2008/132727

(56) References cited:
- EP-A- 0 805 678
- WO-A-00/32172
- US-A1- 2003 017 203
- US-A1- 2005 143 404

## Description

### FIELD OF THE INVENTION

The present invention relates to oval delivery of therapeutic proteins, polypeptides and peptide and, in particular, to oral delivery of insulin.

### BACKGROUND OF THE INVENTION

The delivery of proteins has gained great interest with the development of the biotechnology sector and the advances in recombinant DNA technology that provided large-scale availability of therapeutic proteins. The low oral bioavailability, however, continues to be a problem for most of the large peptides and proteins. The demand for effective delivery of proteins by the oral route has brought a tremendous thrust in recent years both in the scope and complexity of drug delivery technology.

The important therapeutic proteins and peptides being explored for oral delivery induce insulin, salmon calcitonin, interferon, human growth hormone, glucagons, gonadotropin-releasing hormones, enkephalins, vaccines, enzymes, hormone analog, and enzyme inhibitors.

Several barriers exist to the manufacturing of effective formulations for oral delivery or proteins and polypeptides. The first challenge in the development of such oral formulations is in the manufacture itself because proteins have complex internal structures that define their biological activity. Any disruption in the primary, secondary, tertiary or quaternary structure of a protein can result in its deactivation or considerable decline of its bioactivity. The main variables that affect protein structure and stability are related to the temperature, pH, solvent quality, presence of other solutes and the crystallize states of the protein. These considerations are most pertinent when using polymer-encapsulated formulations. Many of the basic encapsulation methods used in the production of polymer-based protein drug delivery systems can easily disrupt the delicate protein structure rendering the protein, e.g. insulin, inactive.

The most commonly used method for preparing solid protein pharmaceuticals is lyophilization (freeze-drying). However, this process generates a variety of freezing and drying stresses, such as solute concentration, formation of ice crystals, and pH changes that can denature a protein to various degrees.

Among the chemical and enzymatic barriers for oral delivery of protein drugs is their cleavage by proteases. Insulin destruction by proteases begins in the stomach and continues by many different enzymes along the gastrointestinal (GI) tract. Pepsins in the stomach together with acid-induced hydrolysis present significant obstacles that prevent oral delivery of proteins and insulin.

Trypsin, chymotrypsin and carboxypeptidases from the pancreas, located in the small intestinal lumen, are responsible for about 20% of the enzymatic degradation of ingested proteins. The remainder of the degradation occurs at the brush-border membrane (by various peptidases) or within the enterocytes of the intestinal tract. Also, a specific cytosolic enzyme called insulin-degrading enzyme, accomplishes the insulin degradation. The presence of just one or two of these enzymes could lead to complete degradation of a protein drug.

The epithelial layer lining the GI tract is a tightly bound collection of cells with minimal leakage and forms a physical barrier to absorption of proteins. In addition, a layer of sulfated mucopolysaccharides and a layer of mucus consisting of glycoproteins, enzymes, electrolytes and water on top of the epithelial layer present yet another physical barrier to the transport of proteins.

Diabetes mellitus (DM) is a metabolic disorder characterized by hyperglycemia, in which the body does not produce enough, or does not properly use, insulin. The result is that the body does not get the energy it needs and unmetabolized sugar (glucose) builds up in the blood, causing damage to the body and its systems. There are two main forms of diabetes: insulin-dependent diabetes (IDDM) or type 1 diabetes, caused by destruction of the pancreatic beta cells that produce insulin and non - insulin - dependent diabetes (NIDDM) or type 2 diabetes, caused by insulin resistance, particularly in skeletal muscle, adipose tissue and liver. Thus, despite hyperinsulinaemia, there is insufficient insulin to compensate for the insulin resistance and to maintain blood glucose in the desirable range.

Exogenous insulin administered to type 2 diabetes mellitus patients failed to reproduce the glucose homeostasis observed in non-diabetic individuals, mostly because subcutaneous parenteral injections deliver insulin to the peripheral circulation rather than to the portal circulation, and directly to the liver - the physiological route in non-diabetic individuals. Because the liver is the primary site or glucose regulation, it is known that insulin delivered into the portal vein is a major determinant of hepatic glucose production.

Normally, blood glucose concentrations are maintained in relatively narrow range as the liver takes up glucose in the fed state and releases it into circulation in appropriated amounts. Thus, hepatic regulation of glucose (in non-diabetics) is associated with lower insulin concentrations than those required when systemic doses of insulin are administrated to regulate glucose. For this reason, parenteral insulin treatment in DM patients produces a peripheral hyperinsulemia, with insulin reaching liver at much lower concentration than associated with direct portal delivery.

The most commonly employed methods to treat type 1 and type 2 diabetes are administration of parenteral (subcutaneous or intradermal) injections of various types of insulin available in the market. On top of the clinical issues, parenteral insulin treatment involves repeat injections, as well as the need for proper storage of the insulin solutions (under refrigeration). Thus a vehicle that will include solid and stable form of insulin for oral uptake would perfectly address these issues. Such, a system would deliver, the exogenous insulin by the oral route, introducing insulin directly to the liver through portal circulation, in a way that closely mimics what occurs in healthy persons. This administration route would provide the benefit of hepatic activation while avoiding hyperinsulemia and its related complications.

The development of a system for oral delivery of insulin as well as for other polypeptides is a superior alternative treatment to the intradermal injection method, and is a technology of major interest for the pharmaceutical industry. For insulin, as an example, despite the many studies, no successful solution in the form of a vehicle is yet available in the market for oral delivery of insulin in a manner that may replace the application by injection.

In developing oral protein delivery systems with high bioavailability, the following approaches might be most helpful: (1) chemical modification of the protein or peptide leading to compounds that are prodrugs or analogues - the prodrug/analogue approach; (2) use of improved delivery carriers and of absorption enhancers such as surfactants, bile salts, or calcium chelators; (3) use of enzyme inhibitors to lower the proteolytic activity; or (4) dosage form modifications. Clearly, it is essential that these approaches maintain the biological activity of the proteins.

In the pro-drug/analogue approach, the proteins or polypeptides are modified so as to engender oral activity. Chemical modification, such as masking or blocking polar amide bonds and terminal amino and carboxyl groups, primarily brings about an alternation in the physicochemical properties of drugs such as lipophilicity, hydrogen-bonding capacity, charge, molecular size, solubility, configuration, isoelectric point, chemical stability, etc., which are known to affect their membrane permeability, enzyme liability, and affinity to carrier systems.

It has been postulated that some type of noncovalent interaction between proteins or polypeptides drugs and delivery agent molecules may be responsible for efficient drug absorption through the intestinal mucosa. These noncovalent interactions of delivery agents and proteins cause temporary stabilization of partially unfolded conformations of proteins, exposing their hydrophobic side chains. The altered lipid solubility of stabilized conformations, as a result of exposed hydrophobic side chains, permits them to gain access to pores of integral membrane transporter and thus be more absorbable through lipid bilayers. The delivery agent-protein combination, which is held together by weak noncovalent intermolccular forces, is assumed to get separated after membrane transport as a result of dilation ensuring reversion of protein into its biologically effective conformation.

This approach favors placing an emulsion of the protein or polypeptide drug to be administered orally in an enteric-coated capsule, which serves as a macrovehicle. In the case of insulin, the liquid form of the drug in the emulsion form implies a lower stability and additional additives are required in order to stabilize the drug, requiring a higher amount of insulin for reducing the blood glucose concentration (300-600 IU). However, the technology of incorporating the emulsion form in a capsule is complex and expensive.

It was found that the coupling of unstable peptides with sugars does improve both hydrolytic stability and membrane permeation. Indeed, insulin modified with sugars was found to be more resistant to enzymatic hydrolysis and exhibited enhanced membrane permeation.

A promising strategy to overcome the so called 'enzymatic barrier' caused by the cytosolic proteases and peptidases in the GI tract comprises the use of enzyme inhibitors and has gained considerable interest in recent years. However, especially for protein and polypeptide drugs that are administered for a longer duration, the co-administration of enzyme inhibitors remains questionable because of side effects caused by these agents and the interference with the regular digestion process of nutritive proteins.

With regard to the dosage form modification approach, a series of matrix carries systems have been considered for dosing of protein and polypeptide drugs, including nanoparticles, microparticles, and self-assembling molecular superstructures. It has been shown that particles in the sub micrometer range and up to 5 µm can cross the intestinal wall intact.

Most of the methods used for the preparation of multiparticulate delivery systems (microparticles and nanoparticles) are based on an emulsion (simple or multiple), solvent evaporation, or solvent extraction scheme. However, the common drawbacks or these methods are low encapsulation efficiency and reduced bioactivity or insulin or other protein and polypeptide drugs after incorporation into the microparticles. Moreover, the penetrability of these multiparticulate systems to aqueous fluids is a serious concern as it can render them susceptible to problems such as initial burst release and loss of protein protection.

Micelles and vesicles are structures held together by the weak hydrophobic-hydrophilic interactions between the head and tail groups of the molecules; however, they exist only in solution and collapse in dry conditions. Self-assembled molecular superstructures, which can maintain their integrity upon drying, are presently under development.

Vesicular systems, such as liposomes and niosomes, have shown great potential in oral delivery of protein and polypeptide drugs. Their biodegradable and nontoxic nature (due to similarity of construction materials to integral components of biomembranes) and capability to encapsulate both hydrophobic and hydrophilic drugs makes them ideal drug carrier systems. However, a major drawback in using vehicular systems for oral application of protein and polypeptide drugs is their low chemical and physical stability. The vesicular structures get easily degraded or disrupted by bile salts in the GI tract, exposing the incorporated protein or polypeptides drug to a harsh GI environment.

WO 95/34294 discloses a controlled release drug delivery system comprising a drug which is susceptible to enzymatic degradation by enzymes present in the intestinal tract; and a polymeric matrix which undergoes erosion in the gastrointestinal tract comprising a hvdrogel-forming polymer selected from the group consisting or (a) polymers which are themselves capable of enhancing absorption of said drug across the intestinal mucosal tissues and of inhibiting degradation of said drug by intestinal enzymes; and (b) polymers which are not themselves capable of enhancing absorption of said drug across the intestinal mucosal tissues and of inhibiting degradation of said drug by intestinal enzymes; wherein when the matrix comprises a polymer belonging to group (b) the delivery system further comprises an agent which enhances absorption of said drug across the intestinal mucosal tissues and/or an agent which inhibits degradation of said drug by intestinal enzymes and when the matrix comprises a polymer belonging to group (a) the delivery system optionally further comprises an agent which enhances absorption or said drug across the intestinal mucosal tissues and/or an agent which inhibits degradation of said drug by intestinal enzymes. The corresponding US Patent No. 6,692,766 claims a synchronous drug delivery composition comprising a polymeric matrix which comprises: 1) polycarbophil, wherein said polycarbophil is blended with a hydrophobic polymer so as to form an erodible matrix, and 2) a drug, wherein erosion of said erodible matrix permits synchronous release of said drug and said hydrogel polymer. The delivery composition may comprise also an agent that inhibits degradation and/or an agent that enhances absorption.

Attempts have been made to deliver insulin orally using poly(alkyl cyanoacrylate) (Damge et al., 1997) and poly(lactide-coglycolide) (Carino et al., 2000) nanospheres, poly(vinyl alcohol)-gel microspheres with protease inhibitor (Kimura et al., 1996), bioadhesives, like hydroxypropyl cellulose, with permeation enhancers, like sodium salicylate (Mesiha and Sidhom, 1995), permeation enhancers, like bile salt-fatty acid-mixed micelles (Scott-Moncrieff et al., 1994), hydroxypropyl methylcellulose phthalate enteric microspheres with sodium *N*-(8-[2 hydroxy benzoyl] amino) caprylate (SNAC) (Qi and Ping, 2004), and Eudragit S100-coated insulin hard-gelatin capsules with sodium salicylate as a permeation enhancer (Hosny et al., 2002). Eudragit S100 entrapped insulin microspheres for oral delivery have been described recently (Jain et al., 2005).

Poly(alkyl cyanoacrylate) nanospheres without the assistance of surfactants (like poloxamer 188 and deoxycholic acid) or surfactants and miglyol 812 cannot protect insulin against in vivo proteolytic degradation (Damge et al., 1997). Polylactide-coglycolide, being a nonenteric polymer, would have pH-independent release, find the released insulin would be degraded by proteolytic enzymes (Carino et al., 2000). Poly(vinyl alcohol)-gel microspheres also suffer from a similar drawback and, thus, need the protection of a protease inhibitor (Kimura et al., 1996). Hydroxypropyl methylcellulose phthalate dissolves at a pH between 5 and 5.5: thus, it would release insulin in the small intestine itself, where it is degraded by trypsin and chymotrypsin. In fact, insulin-loaded hydroxypropyl methylcellulose phthalate microspheres made by double-emulsion solvent evaporation, given orally with SNAC (a permeation enhancer), have been reported to be weakly hypoglycemic in formal rats compared with an oval insulin solution and SNAC (Qi and Ping, 2004).

Although in the last decades there has been a tremendous effort to develop alternative routes, in particular oral, for the administration of active proteins such as insulin, the various developments reported in the literature did not find their way to the market for various reasons. In many cases, processing or storage of the formulation affected the bioactivity of the protein; in other cases, there has been a difficulty in controlling its absorption and in stabilizing it during passage in the digestive tract.

### SUMMARY OF THE INVENTION

One object of the present invention is to provide a system for oral delivery of protein, polypeptide and peptide drugs.

It is another object of the invention to provide such a system that increases the bioavailability of the protein, polypeptide or peptide drug and provides a fast release of the drug.

It is an additional object of the invention to provide a safety mechanism in which the dose of the protein, polypeptide or peptide drug is insulated by an enteric coating of the capsule and embedding of the formulation in enteric polymer particles such that even if there is a leakage in the capsule, the drug will be still protected and the system will still deliver the drug.

It is a further object of the invention to provide a dried drug form system that is stale at ambient temperature thus avoiding the need to refrigerate during storage. However, it is mandatory to keep the drug in a glassy form, i.e., at low water activity (A_{w}) conditions, for example, between 0.0 a_{w} and 0.45 a_{w}, preferably 0.2 a_{w}.

This is achieved by formulating the protein, polypeptide or peptide drug together with a protease inhibitor and optionally an absorption enhancer, each component separately. The components are embedded each in particles with different contents of enteric polymer, and thus their dissolution rate differs, and these particles are contained within a capsule or tablet also coated with an enteric polymer.

The present invention thus relates to a an enteric coated capsule or tablet for oral delivery of a protein, polypeptide or peptide drug in a dry/solid form, said enteric coated tablet or capsule comprises microparticles of said protein, polypeptide or peptide drug and of a protease inhibitor, wherein said protein, polypeptide or peptide drug microparticles are embedded in an enteric polymer matrix.

The capsule or tablet may further comprise particles of an absorption enhancer. In one embodiment, the protease inhibitor and the absorption enhancer microparticles arc separately embedded in enteric polymer matrices, which may be the same or are different from each other and/or from the enteric polymer matrix in which the protein, polypeptide or peptide drug microparticles are embedded. In another embodiment, the capsule or tablet comprises beads of protease inhibitor-absorption enhancer microparticles embedded or not embedded in an enteric polymer matrix, which may be the same or is different from the enteric polymer matrix in which the protein, polypeptide or peptide drug microparticles are embedded.

In one more preferred embodiment of the present invention, the protein, polypeptide or peptide drug is insulin.

### BRIEF DESCRIPTION OF THE FIGURES

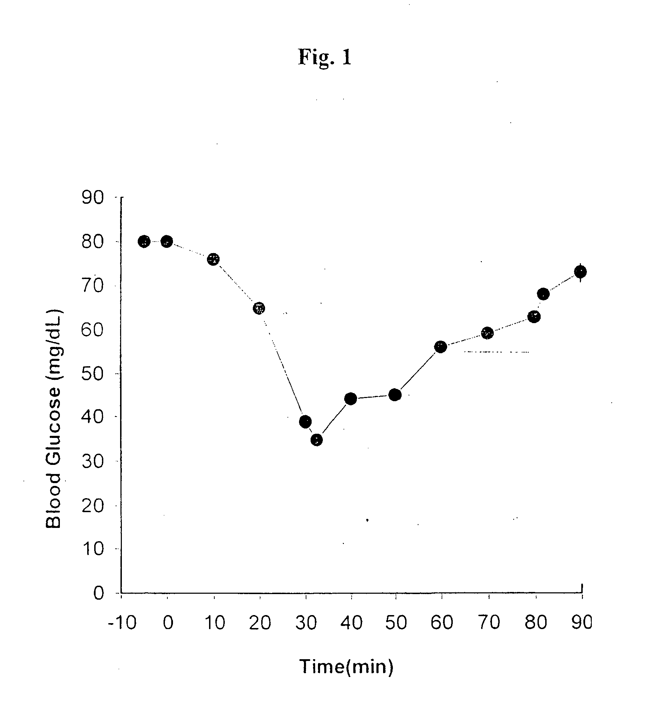

**Fig. 1** shoes blood glucose level in dogs following the administration of an uncoated capsule containing the formulation (insulin, soybean trypsin inhibitor (SBTi) and EDTA) through a cannula to the duodenum.

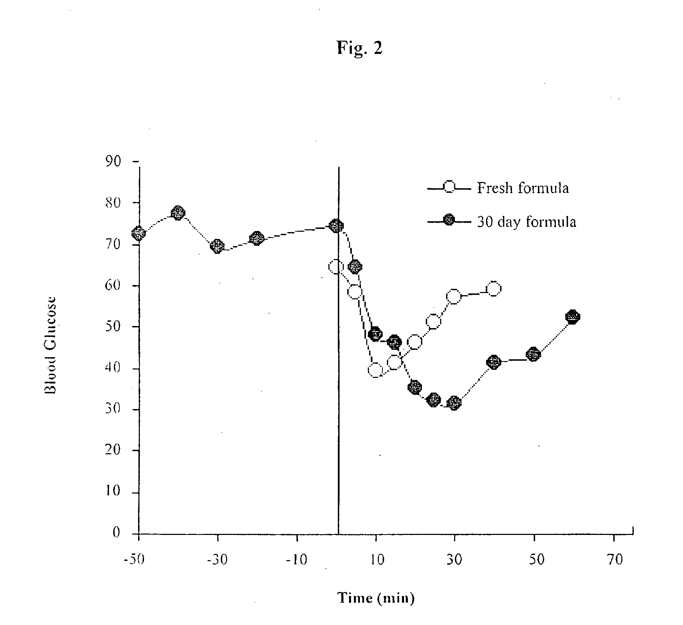

**Fig. 2** is a graph demonstrating the stability of the formulation (insulin, SBTi and EDTA) stored at ambient temperature and very low A_{w} to measure the glassy state or the drug. As can be seen, a similar bioactivity is displayed in the response to the formulation stored at ambient temperature, dissolved in a buffer and injected to the dogs, compared to freshly prepared formulation identically administered. The results show that the formulation is stable at room temperature storage and does not need refrigeration.

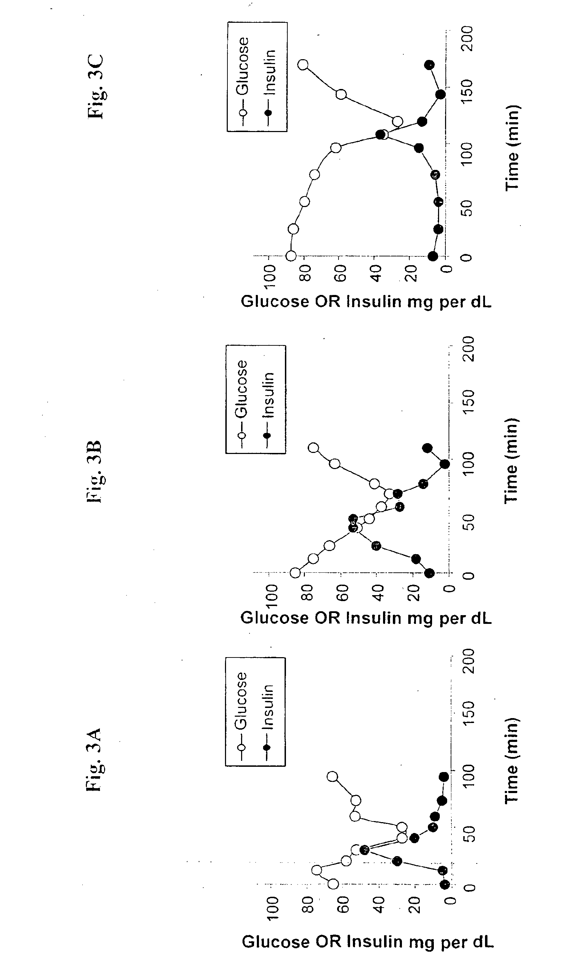

**Figs. 3A-3C** demonstrate glucose and insulin levels in the blood of a fasting dog following the uptake of: **(3A)** uncoated capsule inserted by cannula to the upper duodenum, **(3B)** coated capsule inserted by cannula to the upper duodenum, and **(3C)** coated capsule given orally. The capsules contained insulin and SBTi:EDTA beads and were coated with a Eudragit coating.

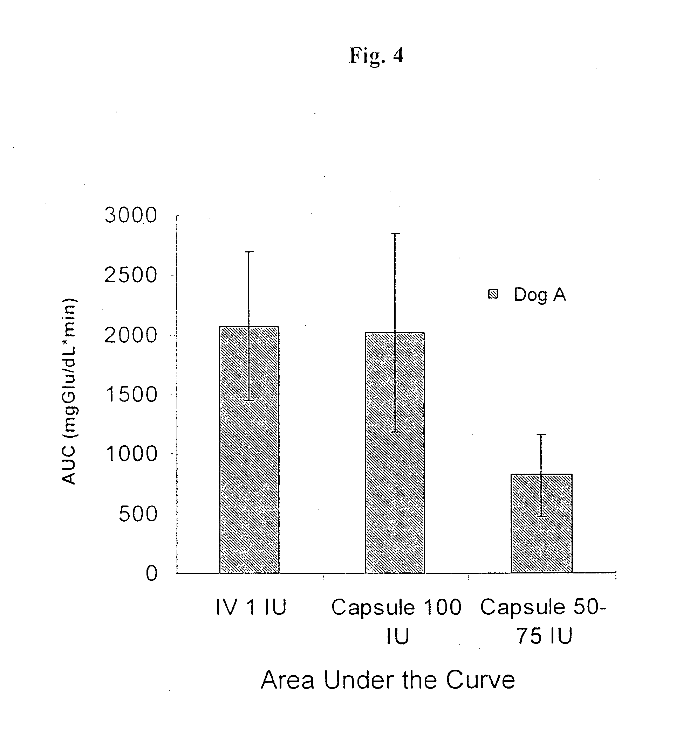

**Fig. 4** shows the area under curve (AUC) of glucose levels showing dose response to the amount of insulin and the administration method: (from left to right) 1 IU insulin intravenous, 100 IU insulin in coated capsule inserted via cannula to the duodenum, and 50-75 IU insulin in coated capsule inserted via cannula to the duodenum. The capsules contained insulin and SBTi:EDTA beads and were coated with is Eudragit coating.

### DETAILED DESCRIPTION OF THE INVENTION

The present invention relates to a solid dosage form coated with an enteric polymer, preferably an enteric coated capsule or tablet for oral delivery of a protein, polypeptide or peptide drug, said enteric coated tablet or capsule comprising microparticles of said protein, polypeptide or peptide drug and of a protease inhibitor, wherein said protein, polypeptide or peptide drug microparticles are embedded in an enteric polymer matrix.

Any protein, polypeptide or peptide drug candidate for oral delivery can be the active component according to the invention such as, but not limited to, insulin, human growth hormone, calcitonin (e.g., salmon calcitonin), an interferon such as an α-, β-, or γ-interferon, glucagon, gonadotropin-releasing hormone, enkephalins, vaccines, enzymes, hormone analogs, and enzyme inhibitors. Preferably, the polypeptide is insulin. In one preferred embodiment, the insulin is human recombinant insulin; in another embodiment, the insulin is of non-human origin such as porcine insulin, that may be natural or recombinant.

Any suitable protease inhibitor may be used in the invention. Preferably the protease inhibitor is a trypsin inhibitor such as soybean trypsin inhibitor (SBTi), but other pretease inhibitors such as, but not limited to, pepstatin, aprotinin, captopril, amasiatin, belastalin, chymostatin, and phosphoramidon may be used.

The formulation may also contain an absorption enhancer that enhances intestinal drug absorption such as, but not limited to, ethylene diamine tetraacetic acid (EDTA), a nonionic chelator; a surfactant such as, but not limited to, a polyoxyethylene ether, sodium laurylsulfate, and a quaternary ammonium compound; a bile salt such as, but not limited to, sodium cholate, sodium deoxycholate or sodium taurodihydrofusidate (STDHF); medium-chain fatty acids such as, but not limited to, caprylic acid, capric acid and lauric acid; medium chain glycerides that may be mono-, di- or tri-glycerides such as, but not limited to, monocaprin, dicaprin and tricaprin or monolaurin, dilaurin and trilaurin; an enamine such as, but not limited to, DL-phenylalanine ethylaceroacetate enamine; a phenothiazine such as chlorpromazine; saponins such as Concanavalin A; sodium salicylate and others.

Enteric polymers for use in the present invention include, but are not limited to, polymer selected from polyacrylates and copolymers thereof, polymethacrylates and copolymers thereof, starches and derivatives thereof, cellulose and derivatives thereof such as ethylcellulose, hydroxypropylmethylcellulose (HPMC), cellulose acetate phthalate (CAP) and hydroxypropyl methylcellulose acetate succinate (HPMCAS), and vinyl polymers such as polyvinyl acetate phthalate (PVAP).

In one preferred embodiment, the enteric polymer is a polymethacrylate copolymer, preferably a copolymer of methacrylic acid with alkyl acrylates and alkyl methacrylates, more preferably a methacrylic acid-ethyl acrylate copolymer such as an Eudragit L30D polymer, or a methacrylic acid-methyl methacrylate copolymer such as an Eudragit L100 (1:1 copolymer) or an Eudragit S100 (1:2 copolymer) polymer, or combinations thereof. In the case of insulin, the most preferred polymers is Eudragit L30 D55 (Degussa, Rohm America), a copolymer dispersion of methacrylic acid and ethyl acrylate at 30% solids.

It is very important that the polymer exhibits dissolution at a pH above the isoelectric point (pl) of the protein. In the particular case of insulin, the pI is 5.4-5.6 and th preferred enteric polymer, Eudragit L30D55, solubilizes at a pH above 5.5.

In one embodiment of the invention, the formulation inside the enteric-coated capsule or tablet comprises microparticles of a protein, polypeptide or peptide drug embedded in an enteric polymer matrix and microparticles of a protease inhibitor that are not embedded in an enteric polymer matrix.

In another embodiment, the formulation inside the enteric coated capsule or tablet comprises protein, polypeptide or peptide drug microparticles embedded in an enteric polymer matrix and protease inhibitor microparticles that are also embedded in an enteric polymers matrix, which may be identical to, or different from, the enteric polymer matrix in which the protein, polypeptide or peptide drug microparticles are embedded.

The formulation inside the enteric-coated capsule or tablet may further comprise microparticles of an absorption enhancer, which may or may not be embedded in an enteric polymer matrix. In a preferred embodiment, the absorption enhancer microparticles are embedded in an enteric polymer matrix, which may be identical to, or different from, the enteric polymer matrix in which the protein, polypeptide or peptide drug microparticles and/or the protease inhibitor microparticles are embedded.

In one preferred embodiment of the invention, the formulation in the enteric-coated capsule or tablet comprises microparticles of the protease inhibitor together with the absorption enhancer, which may or may not be embedded in an enteric polymer matrix. In a preferred embodiment, the microparticles containing the protease inhibitor together with the absorption enhancer are embedded in an enteric polymer matrix, which may be identical to or different from the enteric polymer matrix in which the protein, polypeptide or peptide drug microparticles are embedded, Thus, as shown in the examples herein, microparticles of SBTi together with EDTA or sodium cholate were prepared with and without an enteric polymer matrix. The terms "absorption enhancer" and "penetration enhancer" are used herein interchangeably.

The enteric polymer used for coating the tablet or capsule and the enteric polymers used for embedding each of the components (i.e., protein, polypeptide or peptide drug, protease inhibitor and absorption enhancer) may be the same or different. In one preferred embodiment, they are the same. However, the enteric coating of the capsule or tablet is carried out with a dispersion containing the enteric polymer usually with a plasticizer and a pigment as known in the art. Examples of suitable plasticizers are phthalates and citrates, for example, triethyl citrate, or polyethylene glycol (PEG).

One main characteristic of the enteric-coated capsule or tablet of the invention is that they allow the microparticles of each of the components, due to their porosivc structure, to be fast released at different times at the specific loci in the gastrointestinal tract. This is different from the prior art slow release concept proposed for similar drugs. It is very important to timely release the drug components at specific sequence and time in the duodenum environment (e.g., at the end of the duodenum - the beginning of the jejunum). In order to achieve this effect, the enteric coating of the vehicle (the coated tablet or capsule) has to disintegrate at the chosen loci as function of the pH solubility of the enteric coating and the thickness of its coating (i.e., the weight per unit area or coating weight). The microparticles (e.g., microspheres, beads) size (preferably 02-2.0 mm), the drug-matrix ratio (1:2 to 1:20, preferably 1:2, 1:5, 1:10, 1:15 and up to 1:20) and the temperature (preferably from -35°C to +25°C) of the shelves during freeze drying of the beads also affect the solubility rate and stability of the drug as well as its bioactivity. The solubility rate can be further tuned by the physical conditions used to prepare the microparticles, e.g., buffer strength that affects the final phosphate concentration in the dry formulation, freeze-drying rate (size) and temperature (shelf temperature), which affect porosity.

The present invention provides a platform for oral delivery of peptides and proteins based on a macrovehicle such as a capsule or a tablet coated by an enteric coming polymer. The enteric-coated macrovehicle is targeted to release its content rapidly in the upper part of the duodenum at a pH higher than the protein drug pI (pH 6.3-6.5, which is the intestine pH). It is important for any peptide/protein drug that the dissolution of the enteric polymer (both the enteric polymer coating the macrovehicle and the enteric polymer(s) in which the microparticles are embedded) will occur at a pH higher than the stomach pH and different than the peptide/protein pI. It this way, even if the macrovehicle coating is damaged in the stomach, there is double safety that ensures the safe transport and arrival of the microparticles with the drug past the stomach.

The enteric-coated vehicle contains microparticles, e.g., microspheres embedding the protein, polypeptide or peptide drug, e.g., insulin, and an enzyme inhibitor (that prevents the protein or (poly)peptide destruction by proteases) and optionally a penetration/absorption enhancer (that modulates the transcellular and paracellular pathways of the epithelial layer) for molecular transportation of the protein or (poly)peptide (insulin) from the intestinal lumen to the blood stream. The microparticles matrix is based on acrylic polymer enteric materials. The macrovehicle coating may be of the same acrylic polymer used for the matrix or of a different enteric polymer.

The solid microparticles of the protein or polypeptide drug are obtained by the technology called "spray-freeze-drying" (SFD), which combines processing steps common to freeze-drying and to spray-drying. The protein drug is dissolved, the solution is sprayed into a cryogenic medium, e.g., liquid nitrogen, thus forming a dispersion of frozen droplets, which is then dried in a lyophilizer.

The processing parameters of SFD have an influence on the size and morphology of the produced microparticles and can be manipulated to obtain the desired microparticles. The method can be performed at atmospheric pressure generating drops of desired size and size distribution by a special designed extrusion nozzle, or by extrusion of the matrix wall-drug solution under pressure bellow the level of the cryogenic liquid. The size of the particles will affect the porosity and solubility of the beads. The solubility rate is further tuned by the method used to prepare the microparticles (e.g. buffer strength that affects final phosphate concentration in the dry formula, freeze drying rate and shelves' temperature, which affects porosity. According to the invention, microspheres of diameter ∼50-2000 µm were produced.

In one most preferred embodiment of the invention, an oral insulin drug system was designed whereby enteric-coated capsules containing insulin microparticles and protease inhibitor and absorption enhancer microparticles embedded in an enteric polymer matrix were produced. Experiments with uncoated and coated capsules were performed by inserting them via a cannula to the duodenum of dogs or by oral administration of coated vehicles. In this way, since the vehicle releases the drug very fast in the duodenum, the enzyme inhibitor and the absorption enhancer enable the insulin to reach the portal vein and the liver. The bioactivity of the insulin was determined in the dried microspheres form and in animal blood samples collected at constant time sequences, from the blood stream of treated dogs. The insulin bioavailability was determined according to the decrease in the glucose concentration as function of time.

The process and the formulations were optimized using a bioactivity assay for insulin. Some of the critical points for optimization included: (i) appropriate type and thickness of the enterocoated macrovehicle to release the microspheres at a specific site in the duodenum; (ii) achieving the desired coating:core ratio in the microspheres, in order to obtain a timely release of each component; and (iii) obtaining maximal insulin efficacy (calculated as the ratio (%) of insulin absorbed in the blood relative to a parenteral route). Timely dissolution is achieved also by controlling the particle size or the core:matrix ratio. Examples of the preferred ratios in the present case are 1:2, 1:5, 1:10 and up to 1:20. The fast dissolution is achieved by preparing the microparticles by the spray-freeze-drying technique, which forms a porous matrix with an overall fast dissolution rate.

The concept behind the design of the delivery system of the invention is to have a timely release of each component in a manner that prevents degradation of the protein, polypeptides or peptide drug, e.g. insulin, in the GI, and optimize its absorption once it is dissolved. For this purpose, the drug formulation includes, besides the protein, polypeptide or peptide drug, a protease inhibitor and an absorption enhancer. Each one of the formulation components is embedded separately or in combination with another component in a matrix of an enteric coating material, preferably from the polyacrylate family, in form of microparticles, preferably, microspheres (0.3-1mm diameter). The microspheres are lyophilized according to the SFD technology and, in this way, the formulation compounds and the insulin "core'' and their matrix are in a glassy stable form, thus minimizing their mobility. Finally, the suitable ratios of microspheres are introduced into a macrovehicle, e.g., a capsule, coated by suitable enteric coating materials. The macrovehicle is prepared in a low activity environment.

The acrylic polymer matrix has a three-fold role: (i) to increase the drug, e.g., insulin, stability in aqueous solution form before and during the lyophilization process; (ii) to create a double-enteric protection to the insulin and the active ingredients, in addition to the enteric-coated macrovehicle (tablet or capsule), during their passage and residence in the acidic environment of the stomach; and (iii) to maintain the activity of the drug at ambient temperature.

The macrovehicle is designed to release the active ingredients at an optimum locus in the duodenum (pH 6.3-6.5) at a pH far enough from the insulin pI (5.6), ensuring appropriate environmental conditions of insulin high solubility. The drug components are released rapidly in a controlled kinetic mode and specific sequence based on our experimental results herein. Most importantly, the insulin enters the blood circulation through the hepatic portal vein, the natural way it is administrated by the β-cells of the pancreatic Langerhans islets.

Thus, the technology of the present invention provides a solid form for oral delivery of insulin by which the insulin and other sensitive compounds of the formulation are protected during their solidification by employing the SFD lyophilization technology, the drug formulation is protected against the harsh acid environment in the stomach by creating a double coating defense using a microparticles technology, and the rapid release in a controlled timely manner of the formulation ingredients is obtained by modulating the physical properties of the microspheres (glassy state, porosity, and solubility).

The present invention thus provides a platform for delivery of bioactive proteins to the Gf, with insulin as the leading proof of principle. The concept behind this delivery system provides a delivery formula, which is stable during storage at ambient temperature, and provides a very fast release of the insulin in the small intestine. The careful timely release of the insulin and its assisting protease inhibitors and penetration enhancers ensures high efficacy of the enteric-coated capsule. The use of this form of dry encapsulated proteins and the timely release of their protecting agents can be applied in delivery systems of other bioactive proteins.

The following examples illustrate certain features of the present invention but are not intended to limit the scope of the present invention.

### EXAMPLES

### Example 1. Spray freeze-dried EDTA/SBTi beads.

Materials: SBTi type II-S (Trypsin inhibitor from *Glycine max* (soybean), Sigma-Aldrich, Saint Louis, MO, USA); 5% (w/v) EDTA solution (Sigma-Aldrich, Saint Louis, MO, USA); PBS (phosphate- buffered saline, 0.2M, pH 7.2).

A solution of SBTi type II-S in PBS (1.5 ml for 100 mg SBTi) was prepared by stirring with Tellon^{®}-coated magnetic stirrer until a clear yellow solution was obtained. EDTA solution (5% w/v; 1.5 ml containing 75 mg EDTA for 100 mg of STBi) ways added. The pH level was adjusted to 7.2 with PBS. The EDTA/SBTi solution was injected at a constant flow of 0.4 ml/min to a pneumatic nozzle (Nisco Encapsulation Unit Var JI SPA00336, Nisco Engineering Inc., Zurich, Switzerland), which created droplets of 600 µ-1500 µ in diameter depending on air velocity. The droplets fell into an isolated bowl, containing liquid N₂ (-196°C), and immediately froze to form solid beads. The frozen beads were placed in a freeze drier. After 48 hours, the samples were taken out of the freeze drier and placed in glass vials in a desiccator with a dry environment.

The freeze driers used were with either controlled temperature shelves (Type 3052 + 3060, Secfroid, Lausanne, Switzerland) or with no cooled shelves (Christ Alpha 1-4, Martin Christ Gefriertrocknungsanlagen GmbH., 37507 Ostlrode Am Hartz, Germany). In the freeze drier with controlled temperature shelves, the shelves are cooled to -30°C, the samples placed in aluminum plates on the shelves, and the vacuum is built up to 0.5 mbar, when then the cooling of the shelves is stopped and the water is sublimated.

In the freeze drier with no cooled shelves, the shelves are manually cooled by pouring liquid N₂ on them, the samples are placed on the shelves, and the vacuum is built up to 0.05 mbar. The temperature slowly rises and the water is sublimated.

### Example 2. Spray freeze-dried insulin beads.

**Materials:** Eudragit® L30 D55 (Degussa Rohm Pharma Polymers, Rohm GmbH & Co. KG-Kirschenallee, Darmstadt, Germany); human recombinant insulin Actrapid® (Novo Nordisk, Denmark); PBS (0.2M, pH 7.2).

To an Eudragit^{®} L30 D55 aqueous suspension (pH 2.6), an equal amount in weight of NaOH IN was added to bring the pH value closer to the physiological value. The NaOH created a gel, which was broken to a viscous solution due to an aggressive stirring with a Teflon-coated magnetic stirrer. PBS was added to bring the solution pH to a physiological pH and to lower the viscosity of the solution. Alter obtaining a clear solution with pH 7.2, insulin solution (100 IU=3.5 mg insulin lor 35 mg Eudragit) was added and the solution was stirred mildly. The new insulin solution was drawn with a syringe that was placed in a syringe pump, which controller the flow rate of the solution. The insulin solution was injected at a constant flow of 0.4 ml/min to a pneumatic nozzle, which created droplets of 600µ-1500µ in diameter depending on air velocity. The droplets fell to an isolated bowl containing liquid N₂ (-196°C) and immediately froze to form solid beads. The frozen bends were placed in a freeze drier as in Example 1. After 48 hours the samples were taken out of the freeze drier and placed in glass vials in a desiccator with a dry environment.

### Example 3. Preparation of Insulin-Eudragit beads |N.I.S1]

**Materials:** A solution was prepared to compose of: Insulin (Actrapid® (Novo Nordisk, Denmark): 3-7% (weight/weight); Eudragit L30D55: 80-30%; PBS 0.2M: 0-40%; NaOH 1N: 1-2%. Whenever Eudragit is mentioned below, it is meant to refer to Eudragit L30 D55.

Preparation of the solution: To Eudragit aqueous suspension in a beaker, NaOH (IN) solution was added (150% weight of Eudragit weight), followed by PBS to adjust the pH to 7.2 (about 6.5 the volume of NaOH). Insulin was added and the solution was sprayed into liquid N₂ (-196°C) to form beads. The size of the beads varied from 400 µm - 2000 µm. The beads were dried in a lyophilizer for 48 hours, with shelf temperature of 20°C and down to -30°C. The resulting beads have a ratio of insulin:Eudragit from 1:5 and up to 1:20 (dry matter). The dissolution times of the heads range from 30 sec up to 360 sec. The dissolution time depends on the drying shelf temperature.

### Example 4. Preparation of EDTA-SBTi beards

**Materials:** SBTi: 25-30%; EDTA: 40-45%; Eudragit L30 D55: 0-40%; PBS 0.2M: 25-35%.

SBTi was dissolved in PBS (1 ml/80 mg) and EDTA solution 5% was added (1.2 ml /80mg SBTi). The solution was sprayed into liquid N₂ (-196°C) to form beads. The size of the beads varied from 400 µm - 2000 µm. The beads were dried in a lyophilizer for 48 hours, with shelf temperature of 20°C and down to -30°C. The dry beads dissolved immediately in aqueous solution.

The beads can also be produced with Eudragit, in the same way as described for insulin in Example 3 above. With Eudragit, the dissolution time in water was 20-60 sec.

### Example 5. Sodium Cholate : SBTi Beads

**Materials:** Sodium cholate: 13-17% (Sigma-Aldrich, Saint Louis, MO, USA); SBTi: 10-14%; PBS 0.2M: 25-35%; Eudragit L30D55: 0-30%

SBTi and sodium cholate were mixed at a ratio of 4:5 (SBTi:Cholate). The mixture was dissolved in PBS pH-7.2 and sprayed into liquid N₂ (-196°C) to form beads. The size of the beads varied from 400 µm - 2000 µm. The beads were dried in a lyophilizer for 48 hours, with shelf temperature of 20°C and down to -30°C. The dry beads dissolved immediately in aqueous solution.

The beads can be produced also with Eudragit, in the same way that insulin solution was prepared in Example 3.

### Example 6. Coating of the capsule by film in a coating pan

The apparatus for currying out the coating consists of a coating pan, a portable air supply and exhaust system, and a compressor to generate the spray air. Room air is cleaned by filters, heated and regulated by a valve. The air helps to spray the lacquer suspension and also to open and close the spray gun.

Capsules (approx. 10.0 kg) comprising a mixture of microparticles, containing insulin and others containing protease inhibitor and penetration enhancer are coated in a coating pan apparatus by a coating formulation comprising a coating-pigment dispersion containing, for example, Eudragit L30 (30% dispersion): 333g, 5.6%; pigment (30% suspension): 900 g. 15% ; triethyl citrate : 20 g, 1.1% ; and water: 547 g, 78.3%.

The pigment suspension contains, for example: talc: 49 g, 4.9%; titanium dioxide: 80 g, 8.0%: yellow lake ZLT 3:40g, 4.0%: carbowax 6000: 30 g, 3.0%; silicone antifoam emulsion: 5 g, 0.1%; and water to complete the volume to 1 liter. For the coating, 1% substance (suspension containing a total of 100 g dry lacquer substance, namely the dried dispersion of above) is used for a capsule mass of 10 kg.

Triethyl citrate is dissolved in water and mixed with Eudragit dispersion. Then the pigment suspension is slowly added with continued stirring.

Dust is removed from the capsules, and the capsules are pre-warmed to 30-35°C. The spray suspension is fed into the gun by the means of a peristaltic pump. The spray gun is aimed at the falling cores in the upper part of the pan and the fine jet is sprayed on; at a pressure of 1.5 bar. The rate of spraying should be 25-30 g/min; the supplied air should be at 50-60°C into the lower part of the pan. The tablets should be kept near room temperature. Spraying time should be about 70-100 min. The coated cores are then dried with warm air for about 5 min and sprayed with 50 g of 10% Carbowax solution in water. The tablets are then polished for about 15 min at a reduced speed of pan rotation and without passing warm air. Finally they are blown dry again with warm air. The film-coated tablets are then spread out on a sheet, of filter paper and left to dry for 24 hours at 40°C in a drying room.

### Example 7. In vivo experiment with insulin capsules

Two Beagle-type dogs (Harlan), 10 kg each, with a cannula to the duodenum were used in the experiment.

A catheter is placed in a dog limb vein for drawing blood. Two blood samples were taken before inserting the capsule (or the dissolved formulation) into the dog to determine basal glucose level. A veterinary doctor inserted the capsule through the cannula to the dog's duodenum. When the capsule content was dissolved to form a solution, it was inserted through the cannula with a small funnel and flexible tube. The cannula was closed and the veterinary doctor took a blood sample (∼1 ml) every 5-10 min. The blood was tested for glucose level using a glucometer with disposable glucosticks. Typical glucose response of the dogs is presented in **Fig. 1****.** In this specific experiment, an uncoated capsule containing SBTi:EDTA beads and insulin beads was introduced through the cannula. Insulin beads were prepared as in Example 3, to form beads with Eudragit:Insulin ratio of 10:1. SBTi:EDTA beads were prepared as in Example 4, without Eudragit. Final concentration of the components in each capsule was: Insulin 100 IU, SBTi 80 mg and EDTA 60 mg.

**Fig. 1** demonstrates a marked drop in blood glucose level, which peaked 30 min after application of insulin and the tripsin inhibitor SBTi.

### Example 8: Testing stability of encapsulated Insulin: Eudragit beads

To test the stability of the dry encapsulated formula, insulin microcapsules stored for 1 month at room temperature, were injected to dogs, and the glucose level was monitored as in Example 7. Insulin beads were prepared as in Example 3, to form beads with Eudragit:Insulin ratio of 10:1. After 1 and 30 days, dissolved beads were injected to dogs intravenously (to give dose of 1 IU per dog) and the dogs' blood glucose was monitored as in Example 7. **Fig. 2** shows the stability of the insulin beads and demonstrates that, even after storage of 30 days, encapsulated insulin microparticles are effective in significantly reducing blood glucose levels in a similar manner as freshly prepared insulin microcapsules.

### Examples 9: In-vivo experiments with capsules containing insulin-Endragit and EDTA-SBTi

In-vivo experiments were performed in the same manner as in Example 7, to test additional capsules produced as follows:
1. Capsules filled with insulin-Eudragit beads prepared as described in Example 3 and EDTA:SBTi beads prepared as described in Example 4, uncoated.
2. Capsules as in (1), but coated as described in Example 6.

Insulin beads were prepared as described in Example 3, to form beads with Eudragit:Insulin ratio of 10:1. SBTi:EDTA beads were prepared as described in Example 4, with Eudragit in equal weight to the total weight of SBTi and EDTA together (SBTi:EDTA:Eudragit ratio: 80:100:180). In the preparation of the beads, a manual syringe was used instead of the pneumatic nozzle. Final concentration of the components in each capsule was: Insulin 100 IU, SBTi 80 mg and EDTA 100 mg.

The experiments were performed in the same manner as in Example 7. In a first experiment, the uncoated capsule (J) was introduced via the cannula to the upper duodenum of the dog, in the second the coated capsule (2) was introduced via the cannula to the upper duodenum of the dog, and in the third experiment the coated capsule (2) was administered orally to the dog. Blood was withdrawn as in Example 7, and was tested for glucose levels by a standard glucometer and for insulin using the standard ELISA method. The results in **Fig. 3** show that all capsule provided a drop in glucose level and a peak in blood insulin and prove that the coated capsule is activated only in the upper duodenum.

In another experiment, dogs were administered either intravenously with 1 IU insulin (non-encapsulated nor embedded) or with coated capsule (2) containing either 100 IU or 50-75 IU insulin via the cannula to the upper duodenum. Glucose levels were measured by a standard glucometer and the area under the curve (AUC) was calculated (**Fig. 4**), showing similarity between the results for insulin administered i.v. and for insulin administered in the coated capsule via the duodenum.

### REFERENCES

Carino GP, Jacob JS, Mathiowitz E. Nanosphere based oral insulin delivery. J Control Release. 2000;65:261-269.
Damage C, Vranchx H, Balschmidt P, Couvreur P. Poly(alkyl cyanoacrylate) nanospheres for oral administration of insulin. J Pharm Sci. 1997; 86:1403-1409.
Hosny EA, Al-Shora HI, Elmazar MA. Oral delivery of insulin from enteric coated capsules containing sodium salicylate: effect on relative hypoglycemia of diabetic beagle dogs. Int J Pharm. 2002;237:71-76.
Jain D, Panda AK, Majumdar DK. 2005. Eudragit S100 Entrapped Insulin Microspheres for Oral Delivery. AAPS PharmSciTech. 06(01): E100-E107.
Kimura T, Sato K, Sugimoto K, et al. Oral administration of insulin as poly(vinyl alcohol)-gel spheres in diabetic rats. Biol Pharm Bull. 1996:19:897-900.
Mesiha M, Sidhom M. Increased oral absorption enhancement of insulin by medium viscosity hydroxypropyl) cellulose. Int J Pharm. 1995;114:137-140.
Qi R, Ping QN. Gastrointestinal absorption enhancement of insulin by administration of enteric microspheres and SNAC to rats. J Microencapsul. 2004:21:37-45.
Scott-Mocrieff JC, Shao Z, Mitra AK. Enhancement of intestinal insulin absorption by bile salt-fatty acid mixed micelles in dogs. J Pharm Sci. 1994;83:1465-1469.

## Claims

1. An enteric coated capsule or tablet for oral delivery of a protein, polypeptide or peptide drug comprising microparticles of said drug and of a protease inhibitor, wherein said protein, polypeptide or peptide drug microparticles are embedded in an enteric polymer matrix and the microparticles of said protease inhibitor are optionally embedded in an enteric polymer matrix.

2. The enteric coated capsule or tablet according to claim 1, wherein said protease inhibitor microparticles are embedded in an enteric polymer matrix identical to the enteric polymer matrix in which the protein, polypeptide or peptide drug microparticles are embedded.

3. The enteric coated capsule or tablet according to claim 1, wherein said protease inhibitor microparticles are embedded in an enteric polymer matrix different from the enteric polymer matrix in which the protein, polypeptide or peptide drug microparticles are embedded.

4. The enteric coated capsule or tablet according to any one of claims 1 to 3, further comprising microparticles of an absorption enhancer, wherein said absorption enhancer microparticles are embedded in an enteric polymer matrix, which may be identical or different from the enteric polymer matrix in which the protein, polypeptide or peptide drug microparticles and/or the protease inhibitor microparticles arc embedded.

5. The enteric coated capsule or tablet according to claim 1, comprising microparticles of said protease inhibitor together with an absorption enhancer, wherein said microparticles are embedded in an enteric polymer matrix, which may be identical or different from the enteric polymer matrix in which the protein, polypeptides or peptides drug microparticles are embedded.

6. The enteric coated capsule or tablet according to claim 4 or 5, wherein the protein, polypeptide or peptide drug microparticles, the protease inhibitor microparticles and the absorption enhancer microparticles or the protein, polypeptide or peptide drug microparticles and the protease inhibitor-absorption enhancer microparticles, respectively, are fast released at different times at specific loci in the gastrointestinal tract.

7. The enteric coated capsule or tablet according to claim 1, wherein the enteric polymer used for coating the tablet or capsule and the enteric polymers used for embedding each of the components are different.

8. The enteric coated capsule or tablet according to claim 1, wherein the enteric polymer used for coating the tablet or capsule and the enteric polymers used for embedding each of the components are identical.

9. The enteric coated capsule or tablet according to claim 1, wherein the enteric polymer is selected from polyacrylates and copolymers thereof, polymethacrylates and copolymers thereof, preferably a polymethacrylate copolymer, more preferably a copolymer of methacrylic acid with alkyl acrylates and alkyl methacrylates, most preferably a methacrylic acid-ethyl acrylate copolymer (1:1) (Eudragit), starches and derivatives thereof, cellulose and derivatives thereof such as ethylcellulose, hydroxypropylmethylcellulose (HPMC), cellulose acetate phthalate (CAP) and hydroxypropyl methylcellulose acetate succinate (HPMCAS), and vinyl polymers such as polyvinyl acetate phthalate (PVAP).

10. The enteric coated capsule or tablet according to claim 1, wherein said protein, polypeptide or peptide drug is human growth hormone, calcitonin, interferons, glucagons, gonadotropin-releasing hormones, enkephalins, vaccines, enzymes, hormone analogs, enzyme inhibitors, or, preferably, insulin.

11. The enteric coated capsule or tablet according to claim 1, wherein said protease inhibitor is pepstatin, aprotinin, captopril, amastatin, betastatin, chemostatin, phosphoramidon, or preferably, SBTi (soybean trypsin inhibitor).

12. The enteric coated capsule or tablet according to claim 4 or 5, wherein said absorption enhancer is surfactants, bile salts such as sodium cholate and sodium taurodihydrofusidate (STDHF), medium chain fatty acids, medium chain glycerides, enamines, phenothiazines, saponins, or preferably, ethylene diamine tetraacetic acid (EDTA) or sodium cholate.

13. An enteric coated capsule according to claim 4, comprising microparticles of insulin, SBTi and either EDTA, or sodium cholate, wherein said microparticles of each of the insulin, SBTi, and either EDTA or sodium cholate components are separately embedded in an enteric polymer matrix.

14. An enteric coated capsule according to claim 5, comprising microparticles of insulin and either SBTi-EDTA, or SBTi-sodium cholate, wherein said microparticles of insulin, and either SBTi-EDTA or SBTi-sodium cholate are separately embedded in an enteric polymer matrix.

15. An enteric coated capsule according to claim 13 or 14, wherein the enteric polymer matrices are made of the methacrylic acid-ethyl acrylate copolymer Eudragit L30 D55 (methacrylic acid-ethyl acrylate copolymer (1:1) dispersion 30%) and the capsule enteric coating is carried out with a Eudragit L30 D55 (methacrylic acid-ethyl acrylate copolymer (1:1) dispersion 30%) dispersion optionally comprising a pigment.

## Patentansprüche

1. Eine Kapsel oder Tablette mit enterischer Beschichtung zur oralen Verabreichung eines Protein-, Polypeptid- oder Peptidarzneistoffs, umfassend Mikropartikel des Arzneistoffes und eines Proteasehemmers, wobei die Protein-, Polypeptid- oder Peptidarzneistoff-Mikropartikel in eine enterische Polymermatrix eingebettet sind und die Mikropartikel des Proteasehemmers gegebenenfalls in eine enterische Polymermatrix eingebettet sind.

2. Die Kapsel oder Tablette mit enterischer Beschichtung nach Anspruch 1, wobei die Proteasehemmer-Mikropartikel in eine enterische Polymermatrix eingebettet sind, die mit der enterischen Polymermatrix, in die die Protein-, Polypeptid- oder Peptidarzneistoff-Mikropartikel eingebettet sind, identisch ist.

3. Die Kapsel oder Tablette mit enterischer Beschichtung nach Anspruch 1, wobei die Proteasehemmer-Mikropartikel in eine enterische Polymermatrix eingebettet sind, die sich von der enterischen Polymermatrix, in die die Protein-, Polypeptid- oder Peptidarzneistoff-Mikropartikel eingebettet sind, unterscheidet.

4. Die Kapsel oder Tablette mit enterischer Beschichtung nach einem der Ansprüche 1 bis 3, weiter umfassend Mikropartikel eines Absorptionsverbesserers, wobei die Absorptionsverbesserer-Mikropartikel in eine enterische Polymermatrix eingebettet sind, die mit der enterischen Polymermatrix, in die die Protein-, Polypeptid- oder Peptidarzneistoff-Mikropartikel und/oder die Proteasehemmer-Mikropartikel eingebettet sind, identisch oder davon verschieden sein kann.

5. Die Kapsel oder Tablette mit enterischer Beschichtung nach Anspruch 1, umfassend Mikropartikel des Proteasehemmers zusammen mit einem Absorptionsverbesserer, wobei die Mikropartikel in eine enterische Polymermatrix, eingebettet sind, die mit der enterischen Polymermatrix, in die die Protein-, Polypeptid- oder PeptidarzneistoffMikroaprtikel eingebettet sind, identisch oder davon verschieden sein kann.

6. Die Kapsel oder Tablette mit enterischer Beschichtung nach Anspruch 4 oder 5, wobei die Protein-, Polypeptid- oder Peptidarzneistoff-Mikropartikel, die Proteasehemmer-Mikropartikel und die Absorptionsverbesserer-Mikropartikel oder die Protein-, Polypeptid- oder Peptidarzneistoff-Mikropartikel und die Proteasehemmer-Absorptionsverbesserer-Mikropartikel jeweils schnell zu unterschiedlichen Zeiten an bestimmten Stellen im Magen-Darm-Trakt freigesetzt werden.

7. Die Kapsel oder Tablette mit enterischer Beschichtung nach Anspruch 1, wobei das enterische Polymer, das zur Beschichtung der Tablette oder Kapsel verwendet wird, und die enterischen Polymere, die zum Einbetten der jeweiligen Komponenten verwendet werden, unterschiedlich sind.

8. Die Kapsel oder Tablette mit enterischer Beschichtung nach Anspruch 1, wobei das enterische Polymer, das zum Beschichten der Tablette oder Kapsel verwendet wird, und die enterischen Polymere, die zum Einbetten der jeweiligen Komponenten verwendet werden, identisch sind.

9. Die Kapsel oder Tablette mit enterischer Beschichtung nach Anspruch 1, wobei das enterische Polymer aus Polyacrylaten und Copolymeren davon, Polymethacrylaten und Copolymeren davon, vorzugsweise einem Polymethacrylat-Copolymer, stärker bevorzugt einem Copolymer von Methacrylsäure mit Alkylacrylaten und Alkylmethacrylaten, am stärksten bevorzugt einem Methacrylsäure-Ethylacrylat-Copolymer (1:1) (Eudragit), Stärken und Derivaten davon, Cellulose und Derivaten davon, wie z.B. Ethylcellulose, Hydroxypropylmethylcellulose (HPMC), Celluloseacetatphthalat (CAP) und Hydroxypropylmethylcelluloseacetatsuccinat (HPMCAS), und Vinylpolymeren, wie z.B. Polyvinylacetatphthalat (PVAP), ausgewählt ist.

10. Die Kapsel oder Tablette mit enterischer Beschichtung nach Anspruch 1, wobei der Protein-, Polypeptid- oder Peptidarzneistoff humanes Wachstumshormon, Calcitonin, Interferone, Glucagone, Gonadotropin-freisetzende Hormone, Enkephaline, Impfstoffe, Enzyme, Hormonanaloga, Enzymhemmer oder vorzugsweise Insulin ist.

11. Die Kapsel oder Tablette mit enterischer Beschichtung nach Anspruch 1, wobei der Proteasehemmer Pepstatin, Aprotinin, Captopril, Amastatin, Betastatin, Chemostatin, Phosphoramidon oder vorzugsweise SBTi (Sojabohnentrypsinhemmer) ist.

12. Die Kapsel oder Tablette mit enterischer Beschichtung nach Anspruch 4 oder 5, wobei der Absorptionsverbesserer- grenzflächenaktive Mittel, Gallensalze, wie z.B. Natriumcholat und Natriumtaurodihydrofusidat (STDHF), mittelkettige Fettsäuren, mittelkettige Glyceride, Enamine, Phenothiazine, Saponine oder vorzugsweise Ethylendiamintetraessigsäure (EDTA) oder Natriumcholat ist.

13. Eine Kapsel mit enterischer Beschichtung nach Anspruch 4, umfassend Mikropartikel von Insulin, SBTi und entweder EDTA oder Natriumcholat, wobei die Mikropartikel der jeweiligen Insulin-, SBTi- und entweder EDTA- oder Natriumcholat-Komponenten getrennt in eine enterische Polymermatrix eingebettet sind.

14. Eine Kapsel mit enterischer Beschichtung nach Anspruch 5, umfassend Mikropartikel von Insulin und entweder SBTi-EDTA oder SBTi-Natriumcholat, wobei die Mikropartikel von Insulin und entweder SBTi-EDTA oder SBTi-Natriumcholat getrennt in eine enterische Polymermatrix eingebettet sind.

15. Eine Kapsel mit enterischer Beschichtung nach Anspruch 13 oder 14, wobei die enterischen Polymermatrizes aus dem Methacrylsäure-Ethylacrylat-Copolymer Eudragit L30 D55 (Methacrylsäure-Ethylacrylat-Copolymer (1:1)-Dispersion 30%) bestehen und die enterische Kapselbeschichtung mit einer Eudragit L30 D55 (Methacrylsäure-Ethylacrylat-Copolymer (1:1)-Dispersion 30%)-Dispersion erfolgt, die gegebenenfalls ein Pigment umfasst.

## Revendications

1. Gélule ou comprimé gastro-résistant(e) pour une administration par voie orale d'un médicament à base de protéine, polypeptide ou peptide comprenant des microparticules dudit médicament et d'un inhibiteur de protéase, dans laquelle/lequel lesdites microparticules de médicament à base de protéine, polypeptide ou peptide sont incorporées dans une matrice de polymère entérique et les microparticules dudit inhibiteur de protéase sont éventuellement incorporées dans une matrice de polymère entérique.

2. Gélule ou comprimé gastro-résistant(e) selon la revendication 1, dans laquelle/lequel lesdites microparticules d'inhibiteur de protéase sont incorporées dans une matrice de polymère entérique identique à la matrice de polymère entérique dans laquelle les microparticules de médicament à base de protéine, polypeptide ou peptide sont incorporées.

3. Gélule ou comprimé gastro-résistant(e) selon la revendication 1, dans laquelle/lequel lesdites microparticules d'inhibiteur de protéase sont incorporées dans une matrice de polymère entérique différente de la matrice de polymère entérique dans laquelle les microparticules de médicament à base de protéine, polypeptide ou peptide sont incorporées.

4. Gélule ou comprimé gastro-résistant(e) selon l'une quelconque des revendications 1 à 3, comprenant en outre des microparticules d'un améliorateur d'absorption, dans laquelle/lequel lesdites microparticules d'améliorateur d'absorption sont incorporées dans une matrice de polymère entérique qui peut être identique ou différente de la matrice de polymère entérique dans laquelle les microparticules de médicament à base de protéine, polypeptide ou peptide et/ou les microparticules d'inhibiteur de protéase sont incorporées.

5. Gélule ou comprimé gastro-résistant(e) selon la revendication 1, comprenant des microparticules dudit inhibiteur de protéase avec un améliorateur d'absorption, dans laquelle/lequel lesdites microparticules sont incorporées dans une matrice de polymère entérique qui peut être identique ou différente de la matrice de polymère entérique dans laquelle les microparticules de médicament à base de protéine, polypeptide ou peptide sont incorporées.

6. Gélule ou comprimé gastro-résistant(e) selon la revendication 4 ou 5, dans laquelle/lequel les microparticules de médicament à base de protéine, polypeptide ou peptide, les microparticules d'inhibiteur de protéase et les microparticules d'améliorateur d'absorption ou les microparticules de médicament à base de protéine, polypeptide ou peptide et les microparticules d'inhibiteur de protéase-améliorateur d'absorption, respectivement sont à libération rapide à différents temps au niveau de locus spécifiques dans le tractus gastro-intestinal.

7. Gélule ou comprimé gastro-résistant(e) selon la revendication 1, dans laquelle/lequel le polymère entérique utilisé pour l'enrobage du comprimé ou de la gélule et les polymères entériques utilisés pour l'incorporation de chacun des composants sont différents.

8. Gélule ou comprimé gastro-résistant(e) selon la revendication 1, dans laquelle/lequel le polymère entérique utilisé pour l'enrobage du comprimé ou de la gélule et les polymères entériques utilisés pour l'incorporation de chacun des composants sont identiques.

9. Gélule ou comprimé gastro-résistant(e) selon la revendication 1, dans laquelle/lequel le polymère entérique est choisi parmi les polyacrylates et les copolymères de ceux-ci, les polyméthacrylates et les copolymères de ceux-ci, de préférence un copolymère de polyméthacrylate, plus préférablement un copolymère d'acide méthacrylique avec des acrylates d'alkyle et des méthacrylates d'alkyle, de manière préférée entre toutes un copolymère d'acide méthacrylique-acrylate d'éthyle (1:1) (Eudragit), des amidons et des dérivés de ceux-ci, de la cellulose et des dérivés de celle-ci tels que l'éthylcellulose, l'hydroxypropylméthylcellulose (HPMC), le phtalate d'acétate de cellulose (CAP) et le succinate d'acétate d'hydroxypropylméthylcellulose (HPMCAS) et les polymères vinyliques tels que le phtalate d'acétate de polyvinyle (PVAP).

10. Gélule ou comprimé gastro-résistant(e) salon la revendication 1, dans laquelle/lequel ledit médicament à base de protéine, polypeptide ou peptide est une hormone de croissance humaine, la calcitonine, les interférons, les glucagons, les hormones libérant la gonadotropine, les encéphalines, les vaccins, les enzymes, les analogues d'hormones, les inhibiteurs enzymatiques ou de préférence l'insuline.

11. Gélule ou comprimé gastro-résistant(e) selon la revendication 1, dans laquelle/lequel ledit inhibiteur de protéase est la pepstatine, l'aprotinine, le captopril, l'amastatine, la bétastatine, la chémostatine, le phosphoramidon ou de préférence le SBTi (inhibiteur de la trypsine de soja).

12. Gélule ou comprimé gastro-résistant(e) selon la revendication 4 ou 5, dans laquelle/lequel ledit améliorateur d'absorption est des tensioactifs, des sels biliaires tels que le cholate de sodium et le taurodihydrofusidate de sodium (STDHF), des acides gras de chaîne moyenne, des glycérides de chaîne moyenne, des énamines, des phénothiazines, des saponines ou de préférence de l'acide éthylène diamine tétraacétique (EDTA) ou du cholate de sodium.

13. Gélule gastro-résistant(e) selon la revendication 4, comprenant des microparticules d'insuline, de SBTi et soit d'EDTA, soit de cholate de sodium, dans laquelle lesdites microparticules de chacun des composants insuline, SBTi et soit EDTA, soit cholate de sodium sont incorporées séparément dans une matrice de polymère entérique.

14. Gélule gastro-résistant(e) selon la revendication 5, comprenant des microparticules d'insuline et soit de SBTi-EDTA, soit de SBTi-cholate de sodium, dans laquelle lesdites microparticules d'insuline et soit de SBTi-EDTA, soit de SBTi-cholate de sodium sont incorporées séparément dans une matrice de polymère entérique.

15. Gélule gastro-résistant(e) selon la revendication 13 ou 14, dans laquelle les matrices de polymère entérique se composent du copolymère d'acide méthacrylique-acrylate d'éthyle Eudragit L30 D55 (dispersion de copolymère d'acide méthacrylique-acrylate d'éthyle (1:1) à 30 %) et l'enrobage entérique de la gélule est réalisé avec une dispersion d'Eudragit L30 D55 (dispersion de copolymère d'acide méthacrylique-acrylate d'éthyle (1:1) à 30 %) comprenant éventuellement un pigment.
